(19) Europäisches Patentamt
European Patent Office
Office européen des brevets

(11) EP 0 816 403 B1

(12) **EUROPÄISCHE PATENTSCHRIFT**

(45) Veröffentlichungstag und Bekanntmachung des Hinweises auf die Patenterteilung:
**06.05.2004 Patentblatt 2004/19**

(51) Int Cl.[7]: **C08F 220/58**, A61K 47/32, A61K 7/48

(21) Anmeldenummer: **97109995.7**

(22) Anmeldetag: **19.06.1997**

# (54) Wasserlösliche oder wasserquellbare Polymerisate

Water-soluble or water-swellable polymers

Polymères solubles ou gonflables dans l'eau

(84) Benannte Vertragsstaaten:
**DE FR GB NL**

(30) Priorität: **28.06.1996 DE 19625810**

(43) Veröffentlichungstag der Anmeldung:
**07.01.1998 Patentblatt 1998/02**

(73) Patentinhaber: **Clariant GmbH**
**65929 Frankfurt am Main (DE)**

(72) Erfinder:
- **Engelhardt, Fritz, Dr.**
**Virginia 23320 (US)**
- **Frenz, Volker, Dr.**
**55246 Mainz-Kostheim (DE)**
- **Stock, Jochen, Dr.**
**55129 Mainz (DE)**
- **Tardi, Aranka**
**63543 Neuberg (DE)**

(56) Entgegenhaltungen:
**EP-A- 0 503 853**
**EP-A- 0 632 057**
**US-A- 5 080 809**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft wasserlösliche oder wasserquellbare Polymerisate auf Basis von 2-Acrylamido-2-methyl-propan-sulfonaten, deren Herstellung und deren Verwendung zur Erhöhung der Viskosität wäßriger Lösungen.

Wasser- oder lösungsmittelhaltige Mehrkomponentensysteme wie Lösungen, Emulsionen oder Suspensionen werden häufig aus ökonomischen oder anwendungstechnischen Gründen oder aus Stabilitätsgründen auf höhere Viskositäten eingestellt, bzw. verdickt.

So kann z. B. durch Erhöhung der Viskosität der externen oder internen Phase von Emulsionen oder Suspensionen erreicht werden, daß die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert. Dies gilt besonders für Hautpflegemittel und pharmazeutische Salben auf der Haut. Bei vielen technischen Produkten wie Tapetenablösern, Abbeizmitteln oder Flugzeugenteisern verhindert die erhöhte Viskosität ein vorzeitiges Abfließen von der zu behandelnden Fläche. Durch die gleichmäßigere Verteilung und verlängerte Einwirkdauer wird so die Wirksamkeit erhöht. Neben den erwähnten anwendungstechnischen Vorteilen bietet die hohe Viskosität solcher Präparate auch weitere Vorteile bei der Herstellung, Verpackung, Abfüllung und Lagerung sowie beim Transport. Insbesondere ist hier aus sicherheitstechnischer Hinsicht die Verdickung saurer Medien von Bedeutung.

Generell sind die rheologischen Eigenschaften bei der Herstellung und/oder Formulierung kosmetischer, pharmazeutischer oder technischer Präparate ein entscheidendes Kriterium für den Einsatz dieser Produkte in der Praxis. Die eingesetzten Verdicker sollen dabei bereits in möglichst geringen Einsatzmengen zu einer ausreichenden Verdickung führen. Es sollen die Farbe und prinzipiellen Eigenschaften des zu verdickenden Mediums nicht verändert werden.

Um die rheologischen Eigenschaften von wäßrigen oder lösungsmittelhaltigen Systemen, Emulsionen, Suspensionen einzustellen, werden in der Fachliteratur eine Vielzahl von unterschiedlichen Systemen angegeben. Bekannt sind beispielsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z. B. Polyvinylalkohole, Polyacrylamide, Polyacrylsäure und verschiedene Salze der Polyacrylsäure, Polyvinylpyrrolidon, Polyvinylmethylether, Polyethylenoxide, Copolymere aus Maleinsäureanhydrid und Vinylmethylether, sowie diverse Mischungen und Copolymerisate aus den o. a. Verbindungen.

Die genannten Verdickungsmittel zeigen jedoch bei der Anwendung vielfältige Nachteile. So sind z. B. die Cellulosederivate bzw. allgemein die auf natürlichen Rohstoffen basierenden Materialien und die daraus resultierenden Formulierungen sehr anfällig gegen Bakterien. Anwendungstechnisch fallen sie zumeist durch die Bildung unangenehmer, "fadenziehender" Gele auf. Fettsäurepolyethylenglykolester neigen in der Gegenwart von Wasser zur Hydrolyse, die dabei entstehenden unlöslichen Fettsäuren verursachen unerwünschte Trübungen. Verdickungsmittel natürlichen Ursprungs (z. B. Agar-Agar oder Traganth) weisen je nach Herkunft stark schwankende Zusammensetzung auf.

Aus der DE-C-1.042.233 ist die Herstellung vernetzter Copolymerisate bekannt, die mindestens 25 Gew.-% einer niederen aliphatischen $\alpha,\beta$-ungesättigten Carbonsäure oder eines Anhydrids einer niederen, aliphatischen ungesättigten Polycarbonsäure, 0 bis 75 Gew.-% eines davon verschiedenen mono-olefinischen Monomeren und 0,1 bis 30 % (bezogen auf das Gewicht des Monomeren) eines Vernetzers einpolymerisiert enthalten. Ein gravierender Nachteil dieses Verfahrens besteht darin, daß es nur zu Polymerisaten mit freien Carboxylgruppen führt. Diese Verbindungen sind nur schlecht wasserlöslich und haben daher eine schlechte Quellfähigkeit. Um hochquellfähige und gut verdikkende Produkte zu erhalten, ist es erforderlich, diese Produkte, die häufig eine zähe kautschukartige Konsistenz haben, mit Alkali zu mazerisieren. Hierbei wird eine Gallerte erhalten, die durch weiteren Wasserzusatz auf die gewünschte Viskosität eingestellt werden kann. Dieses umständliche Verfahren stellt eine erhebliche Beeinträchtigung der Anwendung der beschriebenen Verdickungsmittel dar.

Aufgabe vorliegender Erfindung ist es somit, Verdickungsmittel bereitzustellen, die die oben genannten Nachteile nicht aufweisen.

Die vorliegende Erfindung betrifft wasserlösliche oder wasserquellbare Polymerisate, die in statistischer Verteilung 90 bis 99,99 Gew.-% an Resten der allgemeinen Formel (1)

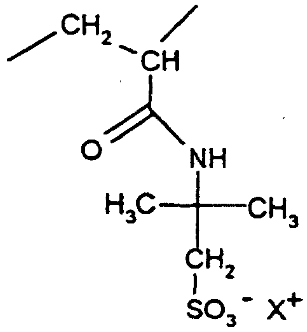

(1)

und 0,01 bis 10 Gew.-% an vernetzenden Strukturen, die aus Monomeren mit mindestens zwei olefinischen Doppelbindungen hervorgegangen sind, enthalten,
wobei $X^+$ für ein Kation oder ein Gemisch von Kationen steht und $X^+$ nicht zu mehr als 10 Mol-% aus Protonen ($H^+$) bestehen darf, und wobei die Anzahl der Reste der allgemeinen Formel (1) im Polymerisat so groß sein muß, daß das hydrodynamische Volumen der Polymerisate in wäßriger Lösung einen Radius von 10 bis 500 nm und eine homogene, unimodale Verteilung aufweist,
dadurch gekennzeichnet, daß man

a.) 90 bis 99,99 Gewichtsteile des 2-Acrylamido-2-methylpropan-sulfonats der allgemeinen Formel (3) in einem Lösungsmittel oder Lösungsmittelgemisch löst oder dispergiert, wobei $X^+$ in diesem Fall auch bis zu 100 Mol-% aus Protonen bestehen kann;

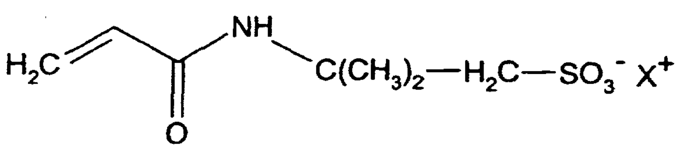

(3)

b.) gegebenenfalls die gemäß a.) erhaltene Lösung oder Dispersion mittels einer oder mehrerer Basen soweit neutralisiert, daß mindestens 90 Mol-% der Sulfonsäure in die Salzform überführt werden;

c.) zu der gemäß a.) und b.) erhaltenen Lösung 0,01 bis 10 Gewichtsteile eines oder mehrerer Vernetzer mit mindestens zwei olefinischen Doppelbindungen zusetzt, und

d.) die Polymerisation in an sich bekannter Weise durch radikalbildende Verbindungen startet und bei einer Temperatur von 10 bis 150°C durchführt,

wobei, die Wahl des in a.) erwähnten Lösungsmittels oder Lösungsmittelgemisches so erfolgt, daß die erhaltenen Polymerisate weitgehend im Lösungsmittel oder Lösungsmittelgemisch unlöslich sind.

Aus der EP 508 853 ist ein Homopolymer aus dem Natriumsalz der Acryloyldimethyltaurinsäure und dem vernetzenden Monomer Methylen-bis-acrylamid bekannt. Dieses Polymer wird jedoch durch Emulsionspolymerisation hergestellt.

Bevorzugte erfindungsgemäße Polymerisate enthalten 98 bis 99,5 Gew.-% an Resten der allgemeinen Formel (1) und 0,5 bis 2 Gew.-% an vernetzenden Strukturen, die aus Monomeren mit mindestes zwei olefinischen Doppelbindungen hervorgegangen sind.
$X^+$ steht insbesondere für ein Proton, ein Kation eines Alkalimetalls, ein Äquivalent eines Kations eines Erdalkalimetalls oder für das Ammoniumion. Bevorzugte erfindungsgemäße Polymerisate sind dadurch gekennzeichnet, daß 90-100 Mol-% der Kationen $X^+$ aus Ammonium-Ionen ($NH_4^+$) und 0 bis 10 Mol-% aus Protonen ($H^+$) bestehen.
Vernetzbare Strukturen, die aus Monomeren mit mindestens zwei olefinischen Doppelbindungen hervorgegangen sind, leiten sich bevorzugt ab von beispielsweise Dipropylenglykol-diallylether, Polyglykoldiallylether, Triethylenglykol-divinylether, Hydrochinon-diallylether, Tetraallyloxyethan oder andere Allyl- oder Vinylether multifunktioneller Alkohole, Tetraethylenglykoldiacrylat, Triallylamin, Trimethylolpropandiallylether, Methylen-bis-acrylamid oder Divinylbenzol.

Besonders bevorzugt leiten sich die vernetzenden Strukturen ab von Monomeren der allgemeinen Formel (2)

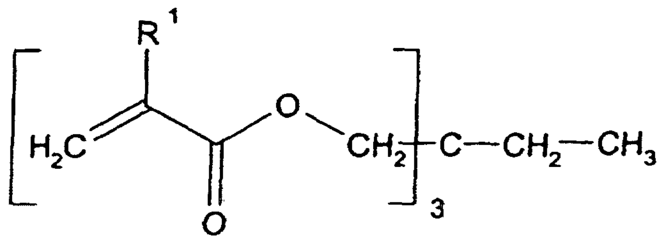

(2)

worin $R^1$ Wasserstoff oder Methyl bedeutet.

Durch die erfindungsgemäß beschriebene Polymerisationsreaktion entstehen neben linearen Ketten verzweigte und vernetzte Polymermoleküle. Diese Moleküle lassen sich durch ihr rheologisches Verhalten in Wasser und durch die dynamische Lichtstreuung charakterisieren.

Rheologisches Verhalten

Das rheologische Verhalten wird durch ein schubspannungskontrolliertes Rheometer bestimmt. Aus den Polymeren wird eine 1 - gewichtsprozentige Lösung hergestellt und die Schubspannung als Funktion der Schergeschwindigkeit ( Scherrate gemessen. Diese Messung erlaubt unmittelbare Rückschlüsse auf die anwendungstechnischen Eigenschaften. Die scherratenabhängige Viskosität errechnet sich als Quotient aus Schubspannung und Schergeschwindigkeit

$$\text{Viskosität} = \frac{\text{Schubspannung}}{\text{Schergeschwindigkeit}}$$

Bei kleinen Scherraten wird die Dimensionsstabilität oder mechanische Festigkeit des ruhenden, nicht beanspruchten Geles gemessen, die hohen Scherraten hingegen korrelieren mit dem mechanischen Verhalten bei starker Beanspruchung wie z.B. beim Auftragen und Verteilen des Geles auf einem Untergrund.

Dynamische Lichtstreuung:

Bei der zweiten Möglichkeit der Charakterisierung durch die dynamische Lichtstreuung wird die Verteilung des hydrodynamischen Volumens der Polymerstrukturen gemessen. Die gelösten Makromoleküle sind flexibel und von einer Solvathülle aus Wassermolekülen umgeben. Bei, wie in diesem Fall, geladenen Polymeren ist die Größe des Makromoleküls stark abhängig vom Salzgehalts des Wassers. Die gleichförmige Ladung entlang der Polymerhauptkette führt in polaren Lösungsmittel zu einer starken Expansion der Polymerkette. Durch die Erhöhung des Salzgehaltes wird der Elektrolytgehalt des Lösungsmittels erhöht und die gleichförmigen Ladungen gegeneinander abgeschirmt.

Neben den in der Solvathülle transportierten Molekülen werden auch Lösungsmittelmoleküle in den Hohlräumen des Polymeren gebunden.

Die Lösungsmittelmoleküle sind dann Teils des Makromoleküls in Lösung und bewegen sich mit derselben mittleren Geschwindigkeit. Das hydrodynamische Volumen beschreibt nun die Lineardimension des Makromoleküls und dieser Solvatmoleküle.

$$V_h = \frac{M}{N_A}(v_2 + dv_1)$$

$V_h$ = hydrodynamisches Volumen
M = Masse des unsolvatisierten Makromoleküls
$N_A$ = Loschmidtsche Konstante
$v_1$ = spezifisches Volumen des Lösungsmittels
$v_2$ = spezifisches Volumen des Makromoleküls
d = Masse Lösungsmittel (in g), die mit einem Gramm unsolvatisiertem Makromolekül verbunden ist

Wenn das hydrodynamische Teilchen Kugelform besitzt, so kann man leicht aus dem hydrodynamischen

Volumen einen hydrodynamischen Radius errechnen:

$$V_h = \frac{4\,\pi\,R^3}{3}$$

R = hydrodynamischer Radius

Ein hydrodynamisches Teilchen ist nur in sehr selten Fällen eine exakte Kugel. Bei den meisten synthetischen Polymeren handelt es sich um Knäuelstrukturen oder Ellipsoide großer Exzentrizität. Die Angabe eines Radius bezieht sich dann auf eine dem Körper reibungsäquivalente Kugel.

Die bisherigen Gleichungen gelten nur für den Fall monodisperser Polymersysteme. In der Praxis erhält man aber immer Verteilungen von Molekulargewichten und damit auch Verteilungen von hydrodynamischen Radien und Volumen. Für polydisperse Systeme muß durch Laplacetransformation der Integralgleichung (2) die Verteilung der Diffusionskoeffizenten errechnet werden.

Aus dieser Verteilung kann man auf die Radienverteilung und die Verteilung der hydrodynamischen Volumen schließen.

Ein bevorzugtes Verfahren zur Herstellung der erfindungsgemäßen Polymerisate ist dadurch gekennzeichnet, daß man

a.) 98 bis 99,5 Gewichtsteile des 2-Acrylamido-2-methylpropan-sulfonats der allgemeinen Formel (3) in einem Lösungsmittel oder Lösungsmittelgemisch löst oder dispergiert, wobei $X^+$ in diesem Fall auch bis zu 100 Mol-% aus Protonen bestehen kann;

b.) gegebenenfalls die gemäß a.) erhaltene Lösung oder Dispersion mittels einer oder mehrerer Basen soweit neutralisiert, daß mindestens 90 Mol-% der Sulfansäure in die Salzform überführt werden;

c.) zu der gemäß a.) und b.) erhaltenen Lösung 0,5 bis 2 Gewichtsteile eines oder mehrerer Vernetzer mit mindestens zwei olefinischen Doppelbindungen zusetzt, und

d.) die Polymerisation in an sich bekannter Weise durch radikalbildende Verbindungen startet und bei einer Temperatur von 10 bis 150°C durchführt,

wobei, die Wahl des in a.) erwähnten Lösungsmittels oder Lösungsmittelgemisches so erfolgt, daß die erhaltenen Polymerisate weitgehend im Lösungsmittel oder Lösungsmittelgemisch unlöslich sind.

Die Polymerisationsreaktion erfolgt vorzugsweise in einem wasserlöslichen Alkohol oder einem Gemisch mehrerer wasserlöslicher Alkohole mit einem bis 4 C-Atomen, vorzugsweise in tert.-Butanol. Der Wassergehalt des Alkohols oder des Gemisches mehrerer Alkohole darf 10 Gew.-% nicht überschreiten, da sonst im Verlauf der Polymerisation Klumpenbildung auftreten kann. Konkret hat die Wahl der Art und der Menge des Lösungsmittels so zu erfolgen, daß die eingesetzte Menge an 2-Acrylamido-2-methylpropansulfonsäure und/oder das zu polymerisierende Salz weitgehend löslich oder dispergierbar ist. Unter weitgehend löslich oder dispergierbar ist zu verstehen, daß sich auch nach Abstellen des Rührwerks kein festes Material aus der Lösung oder Dispersion absetzt. Das im Verlaufe der Reaktion entstehende Polymerisat hingegen soll in dem gewählten Lösungsmittel (oder -gemisch) jedoch weitgehend unlöslich sein. Unter weitgehend unlöslich ist hierbei zu verstehen, daß im Verlauf der Polymerisation eine gut rührbare breiige Polymerpaste entsteht, in der sich keine Klumpen oder Verklebungen bilden dürfen. Das durch Absaugen der Paste erhältliche Filtrat darf einen Feststoffgehalt von maximal 5 Gew.-% aufweisen. Sind die Polymerisate in stärkerem Ausmaß im gewählten Lösungsmittel oder Lösungsmittelgemisch löslich, kann es beim Trocknen der Polymerisatpaste zu Verklumpungen kommen.

Die Polymerisationsreaktion selbst wird in an sich bekannter Art und Weise durch radikalbildende Verbindungen wie Azoinitiatoren (z.B. Azo-bisisobutyronitril), Peroxiden (z.B. Dilauroylperoxid) oder Persulfaten in einem geeigneten Temperaturintervall von 20°C bis 120°C, vorzugsweise zwischen 40°C und 80°C, ausgelöst und über einen Zeitraum von 30 min bis mehreren Stunden fortgeführt.

Beispiel 1:

In einem 5,0 l Quickfitkolben mit Ankerrührer, Rückflußkühler, Innenthermometer, Einleitungsmöglichkeit für $N_2$ und $NH_3$ (gasförmig) werden 2006,2 g tert-Butanol (97,5 %-ig, Rest: Wasser) vorgelegt. Anschtießend werden 340,0 g 2-Acrylamido-2-methyl-propan-sulfonsäure eingetragen und unter starkem Rühren dispergiert, wobei eine

leichte Trübung des Lösungsmittels erhalten bleibt. Über einen Zeitraum von ca. 30 min leitet man nun 27-28 g Ammoniak (gasförmig, etwa äquimolar mit der 2-Acrylamido-2-methyl-propansulfonsäure) in den überstehenden Gasraum ein und rührt weitere 30 min bei Raumtemperatur nach bzw. bis sich ein pH-Wert von 6,0 ± 0,5 eingestellt hat. Man gibt 32,0 g TMPTA-Lösung (Trimethylolpropantriacrylat) (25 %-ig in tert-BuOH) zu und erwärmt auf eine Temperatur von T=60°C, wobei die Reaktionsmischung durch gleichzeitiges Einleiten von Stickstoff inertisiert wird. Nach Erreichen der Temperatur von T=60°C werden 4,0 g DLP (Dilauroylperoxid) zugegeben. Die Reaktion springt unmittelbar nach Zugabe des Initiators an, was an einem Anstieg der Temperatur und am Ausflocken des Polymerisates zu erkennen ist. Etwa 15 min nach dem Einsetzen der Polymerisationsreaktion wird die Stickstoffzufuhr eingestellt. Ungefähr 30 min nach Zugabe des Starters DLP erreicht die Temperatur ein Maximum (ca. 65 - 70°C). Weitere 30 min nach Durchlaufen dieses Maximums wird zum Rückfluß erhitzt und unter diesen Bedingungen zwei Stunden nachgerührt. Der Inhalt des Reaktionsgefäßes nimmt im Verlauf der Reaktion eine breiartige Konsistenz an, ist aber noch gut rührbar. Anschließend wird auf Raumtemperatur abgekühlt und der Feststoff abgesaugt. Die Paste wird bei 60 - 70°C über 24 h im Vakuumtrockenschrank getrocknet. Man erhält 391,0 g eines feinen weißen Pulvers.

Das Pulver wird getrocknet und zu 1,0 Gew.-% in destilliertem Wasser aufgelöst. Dabei entsteht ein festes, klares Gel, das sich hervorragend als Verdicker für wäßrige Lösungen eignet. Die mit diesem Verdicker hergestellten Gele eignen sich insbesondere für kosmetische Anwendungen, da sie beim Verteilen auf dem Körper ein angenehmes Hautgefühl hervorrufen.

Beispiel 2:

Das Beispiel 1 wird wiederholt mit dem Unterschied, daß nach der Neutralisation durch Ammoniak lediglich 19,2 g 25 %-igen TMPTA-Lösung zugegeben werden.

Das rheologische Verhalten derartig hergestellter Gele wird in einem schubspannungs-kontrollierten Rheometer wie folgt bestimmt:

Verwendete Geräte:

1. Schub-spannungs-kontrollierter Rheometer
2. Platte-Platte Meßgeometrie aus Edelstahl (Durchmesser: 20 mm)
3. Infrarottrockner mit:
   - Waage und
   - Schreiber
4. Mörser und Pistill
5. 100 ml Glas mit Schraubverschluß

Probenvorbereitung:

Etwa 1,5 g des pulvrigen Materials werden im Mörser fein zerrieben und 15 min bei 120°C im Infrarotrockner getrocknet. Vom getrockneten Material wird in einem sauberen Glas mit Schraubdeckelverschluß ein genau 1,0 %-iges Gel in deionisiertem Wasser hergestellt:

Einwaage: 0,50 g Probe einwiegen und auffüllen auf 50,00 g Gesamtgewicht mit deionisiertem Wasser

Man läßt die Probe stehen bis ein homogenes optisch transparentes Gel entsteht.

Das Probenmaterial wird in dem Zwischenraum zwischen den Platten des schubspannungskontrollierten Rheometers möglichst gleichmäßig aufgebracht. Befindet sich zu viel oder zu wenig Material im Zwischenraum; sollte die Probe erneut aufgetragen werden. Man gibt nun ein Drehmoment vor, so daß sich eine Scherrate von 5 - 10 $s^{-1}$ einstellt. Typischerweise wird ein Drehmoment von 400 - 600 N benötigt. Bei dieser Drehgeschwindigkeit werden die in der Probe befindlichen Luftblasen herausgeschert. Am Rand der Platte muß man dabei vorsichtig neues Probenmaterial zugeben, so daß sich dort ein senkrechter Meniskus bildet.

Fehlt zwischen den Platten Probenmaterial oder aber ist zuviel vorhanden, kommt es zu einem Abrutschen der Probe an den Platten und einem unstetigen Kurvenverlauf.

In einem Scherratenintervall von etwa 0,01 $s^{-1}$ bis 15 $s^{-1}$ werden die Fließ- und

Viskositätskurve für die Probe aufgenommen. Man führt die Messung an jeder Probe insgesamt dreimal durch,

wobei das Material für alle drei Messungen zwischen den Platten verbleibt.

Alle drei Messungen müssen qualitativ denselben Verlauf aufweisen und dürfen etwa 5 % quantitativ voneinander abweichen.

Zunächst wird die Viskosität bei einer Scherrate von 10 $s^{-1}$ bestimmt. Zur Auswertung wird dabei lediglich der dritte Meßdurchlauf gewählt. Weiterhin wird auch der gesamte Verlauf einer Vikositätskurve herangezogen. Dazu wird auf der Ordinate der dekadische Logarithmus der Viskosität gegen den dekadischen Logarithmus der Scherrate auf getragen.

Für die Beispiele 1 und 2, die sich beide als Verdicker einsetzen lassen, ergibt sich folgendes Meßergebnis:

Ergebnis der rheologischen Untersuchungen:

Meßergebnisse der schubspannungskontrollierten Experimente

| Probenbezeichung | Viskosität bei einer Scherrate von 0.1 Hz in Pa*s | Viskosität bei einer Scherrate von 10 Hz in Pa*s |
|---|---|---|
| Beispiel 1 | 1040 | 30 |
| Beispiel 2 | 312 | 16 |

Der hydrodynamische Radius der Partikel wird durch dynamische Lichtstreuung bestimmt.

Physikalischer Hintergrund:

Die dynamische Lichtstreuung ermittelt die zeitgemittelte Intensitätsautokorrelationsfunktion des gestreuten Lichtes /1,2/:

$$G^{(2)} = \langle |(t,q) / (O,q)| \rangle = A\,[1 + \beta\, |g^{(1)}(q,t)|^2] \qquad (1)$$

wobei A die gemessene Basislinie, β ein Kohärenzparameter, t die Korrelationszeit und q der Streuvektor bedeutet. Die normalisierte Korrelationsfunktion der elektrischen Feldstärke $g^{(1)}(q,t)$ setzt sich in einem polydispersen System aus den Komponenten im Streuvolumen zusammen /3/

$$g^{(1)}(q,t) = \int_0^\infty G(\Gamma)\exp(-\Gamma \cdot t)\,d\Gamma \qquad (2)$$

Aus der Linienbreite $\Gamma$ lassen sich mit Hilfe des Stokes - Einstein - Diffusionskoeffizenten die hydrodynamischen Radien der beteiligten Komponenten errechnen:

$$\frac{\Gamma}{q^2} = D = \frac{kT}{6\pi\eta R} \qquad (3)$$

wobei k die Boltzmann-Konstante, T die absolute Temperatur in Grad Kelvin, η die Viskosität des Lösungsmittels ( Wasser und R der hydrodynamische Radius der beteiligten Komponenten ist.

Die Ermittlung der Linienbreite aus der Feldautokorrelationsfunktion wird mit Hilfe von CONTIN durchgeführt /3,4,5 /

Probenpräparation:

Das Reaktionsprodukt der Fällungspolymerisation wird für 24 Stunden bei 70°C im Vakuumtrockenschrank bis zur Gewichtsbeständigkeit getrocknet. Aus diesem Material wird eine 0.01 gewichtsprozentige Lösung im bidestilliertem Wasser (Leitfähigkeit 15 MΩ /cm) hergestellt. Die Lösung wird dann solange verdünnt, bis die Autokorrelationsfunktion der elektrischen Feldstärke unabhängig von der Konzentration ist. Dieser Zustand wird als unendliche Verdünnung bezeichnet, die Bewegung der Polymere und Polymernetzwerke ist ungestört und diffusiv. Die ermittelte

Autokorrelationsfunktion der elektrischen Feldstärke ist damit nun noch eine Funktion der beteiligten Komponenten und ihrer Verteilung. Alle die oben genannten Gleichungen gelten nur für den Fall der unendlichen Verdünnung nichtwechselwirkender Teilchen.

Apparatur zur dynamischen Lichtstreuung:

Eine kommerziell erhältliche Apparatur zur dynamischen Lichtstreuung /6/ ist wie folgt aufgebaut: (ALV/DLS/SLS 5000 F Monomodefaser Kompakt Goniometer System Version A Dual Plus 532) mit einem kommerziell erhältlichen Multiple-Tau Hardware Korrelator /6/ mit einem Nd-YAG-Festkörperlaser /7/ (ELS Modell 140-0532-100), bei einer Wellenlänge von 532 nm mit einer mittleren Leistung von 110 mW, wird zur Analyse der Teilchengrößenverteilung verwendet. Der eingehende Strahl ist vertikal polarisiert ( 1:100 ) in Bezug zur Streuebene /1/.
Die Analyse der Teilchengrößenverteilung ist nur möglich, wenn β aus (1) einen Wert sehr nahe bei eins besitzt, in unserem Experiment 0.98 /8/. Aufgrund des hohen Kohärenzfaktors β ist eine Analyse sehr verdünnter Proben bei gutem Signal-Rausch-Verhältnis möglich und dies reduziert den Fehler der Extrapolation auf Konzentration Null auf ein Minimum.

Literatur:

/1/ Pecora, R.; *Dynamic Light Scattering;* Plenum Press, New York, 1976
/2/ Chu, B.; *Laser Light Scattering;* Academic Press: New York, 1994
/3/ Schmitz, K.S. *; An introduction to dynamic light scattering by macromolecules;* Academic Press : New York, 1990
/4/ Provincher S. W. *; Comp. Phys.* 27, 213, 1982
/5/ Provincher S. W.; *Comp. Phys.* 27, 229, 1982
/6/ ALV Laservertriebsgesellschaft mbH, Robert Bosch Str. 47, D-63225 Langen, Germany
/7/ ELS - Reinheimer Straße 1 1, D - 64846 Groß - Zimmern, Germany
/8/ Wu, C. et. al.; *Macromolecules*, 1995, 28, 4914 - 4919


Nach dem vorstehend beschriebenen Verfahren wurde für den Hydrodynamischen Radius der Hauptkomponenten der Beispiele 1 und 2, ermittelt durch dynamische Lichtstreuung, die folgenden Werte erhalten:

Beispiel 1: 440 nm

Beispiel 2: 160 nm

Beispiel 3:

Das Beispiel 1 wird wiederholt mit dem Unterschied, daß nach der Neutralisation durch Ammoniak keine Vernetzer-Lösung (TMPTA-Lösung) zugegeben wird.
Die Viskosität einer 1,0 %-igen wäßrigen Lösung von Beispiel 3 zeigt bei einer Scherrate von 10 $s^{-1}$ eine Viskosität von 0,035 Pa•s. Die erzielte Viskosität liegt daher um einen Faktor 100 (bzgl. Beispiel 2) bis 1000 (bzgl. Beispiel 1) niedriger. Derartige Polymerisate sind aufgrund der geringen Viskosität nicht mehr zur effizienten Verdickung von wäßrigen Lösungen, insbesondere zur Herstellung kosmetischer Formulierung geeignet. Zudem neigen die aus solchen unverzweigten, d. h. linearen Polymeren hergestellte Lösungen zum "Fädenziehen", was insbesondere bei der Herstellung von kosmetischen Emulsionen hinderlich ist. Die Messung des hydrodynamischen Volumens ergibt folgendes Ergebnis:

Hydrodynamischer Radius der Hauptkomponente ermittelt durch dynamische Lichtstreuung:

Beispiel 3: 5.5 nm ( lineares Polymer )

Beispiel 4 (Dosiervariante):

160,0 g destilliertes Wasser werden vorgelegt und auf 95°C erhitzt. Innerhalb von zwei Stunden werden nun 221,2 g einer 50 %-igen wäßrigen Lösung eines AMPS-Natrium-Salzes und gleichzeitig (aber aus getrennter Vorlage) 120,0 g einer 1,0 %-igen wäßrigen 2,2'-Azo-bis-amidinopropan-dihydrochlorid-Lösung zugepumpt. Nach Ende der

Zudosierung wird noch eine Stunde unter Rückfluß nachgerührt. Anschließend wird die so erhaltene Lösung getrocknet. Auch diese Probe wird wie weiter oben beschrieben rheologisch geprüft und der hydrodynamische Radius durch Lichtstreuung gemessen.

Die rheologische Untersuchung ergibt folgendes Ergebnis:

Meßergebnisse der schubspannungskontrollierten Experimente

| Probenbezeichung | Viskosität bei einer Scherrate von 0.1 Hz in Pa*s | Viskosität bei einer Scherrate von 10 Hz in Pa*s |
|---|---|---|
| Beispiel 3 | 0.1 | 0.035 |
| Beispiel 4 | 0.25 | 0.009 |

Derartige Materialien sind nicht mehr als Verdicker einsetzbar.

Auch das hydrodynamische Volumen derartiger Polymerisate in Wasser ist viel zu klein.
Hydrodynamischer Radius der Hauptkomponente, ermittelt durch dynamische Lichtstreuung:

Beispiel 4: 7 nm ( lineares Polymer )

Die Überlegenheit der beschriebenen, verzweigten Polymerisate zeigt sich insbesondere beim Verdicken saurer Lösungen. Eine 1 %-ige wäßrige Lösung (in destilliertem Wasser), zeigt eine gelartige Konsistenz. Dies gilt sowohl für Materialien, die gemäß Beispiel 1 oder Beispiel 2 erhalten werden. Handelsübliche Verdicker, die auf Basis von Polymerisaten der Acrylsäure oder deren Derivate aufgebaut sind (z. B. Carbool 980), zeigen nach der oben geschilderten Meßmethode in 1 %-iger wäßriger Lösung im neutralen oder leicht alkalischen Bereich Viskositäten von mehr als 30 Pa•s. Mit sinkendem pH-Wert läßt jedoch deren Verdickungsvermögen (bzw. die gemessene Viskosität) stark nach. Die vorstehend beschriebenen erfindungsgemäßen Materialien können ihre Viskosität jedoch bis zu einem pH-Wert von ca. 3 aufrecht erhalten.

**Patentansprüche**

1. Wasserlösliche oder wasserquellbare Polymerisate, die in statistischer Verteilung 90 bis 99,99 Gew.-% an Resten der allgemeinen Formel (1)

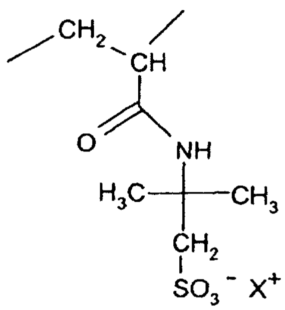

(1)

und 0,01 bis 10 Gew.-% an vernetzenden Strukturen, die aus Monomeren mit mindestens zwei olefinischen Doppelbindungen hervorgegangen sind, enthalten, wobei $X^+$ für ein Kation oder ein Gemisch von Kationen steht und $X^+$ nicht zu mehr als 10 Mol-% aus Protonen ($H^+$) bestehen darf, und wobei die Anzahl der Reste der allgemeinen Formel (1) im Polymerisat so groß sein muß, daß das hydrodynamische Volumen der Polymerisate in wäßriger Lösung einen Radius von 10 bis 500 nm und eine homogene, unimodale Verteilung aufweist, **dadurch gekennzeichnet, daß** man

a.) 90 bis 99,99 Gewichtsteile des 2-Acrylamido-2-methyl-propansulfonats der allgemeinen Formel (3) in einem Lösungsmittel oder Lösungsmittelgemisch löst oder dispergiert, wobei $X^+$ in diesem Fall auch bis zu 100 Mol-% aus Protonen bestehen kann

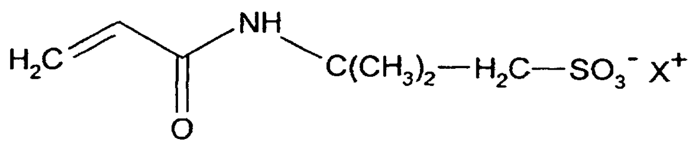

(3)

b.) gegebenenfalls die gemäß a.) erhaltene Lösung oder Dispersion mittels einer oder mehrerer Basen soweit neutralisiert, daß mindestens 90 Mol-% der Sulfonsäure in die Salzform überführt werden;

c.) zu der gemäß a.) und b.) erhaltenen Lösung 0,01 bis 10 Gewichtsteile eines oder mehrerer Vernetzer mit mindestens zwei olefinische Doppelbindungen zusetzt, und

d.) die Polymerisation in an sich bekannter Weise durch radikalbildende Verbindungen startet und bei einer Temperatur von 10 bis 150°C durchführt,

wobei, die Wahl des in a.) erwähnten Lösungsmittels oder Lösungsmittelgemisches so erfolgt, daß die erhaltenen Polymerisate weitgehend im Lösungsmittel oder Lösungsmittelgemisch unlöslich sind.

2. Wasserlösliche oder wasserquellbare Polymerisate gemäß Anspruch 1, **dadurch gekennzeichnet, daß** sie 98 - 99,5 Gew.-% an Resten der allgemeinen Formel (1) und 0,5 bis 2 Gew.-% an vernetzenden Strukturen, die aus Monomeren mit mindestens zwei olefinischen Doppelbindungen hervorgegangen sind, enthalten.

3. Wasserlösliche oder wasserquellbare Polymerisate gemäß Anspruch 1 und/oder Anspruch 2, **dadurch gekennzeichnet, daß** die vernetzenden Strukturen aus Monomeren der allgemeinen Formel (2)

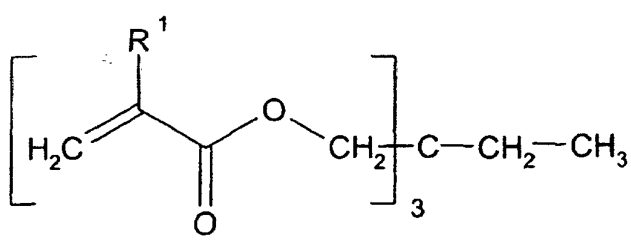

(2)

hervorgegangen sind, in der $R^1$ Wasserstoff oder Methyl bedeuten.

4. Wasserlösliche oder wasserquellbare Polymerisate gemäß einem oder mehreren der Ansprüche 1 bis 3, **dadurch gekennzeichnet, daß** 90 - 100 Mol-% der Kationen $X^+$ aus Ammonium-Ionen ($NH_4^+$) bestehen und 0 bis 10 Mol-% aus Protonen $H^+$.

5. Wasserlösliche oder wasserquellbare Polymerisate gemäß mindestens einem der Ansprüche 1 bis 4, **dadurch gekennzeichnet, daß** man ein Lösungsmittelgemisch aus 90-100 Gewichtsteilen eines oder mehrerer Alkohole und 0-10 Gewichtsteilen an Wasser einsetzt, wobei als Alkohol bevorzugt tert.-Butanol eingesetzt wird.

6. Verwendung der Polymerisate gemäß einem oder mehrerer der Ansprüche 1 bis 5 zum Verdicken von Flüssigkeiten.

7. Verwendung gemäß Anspruch 6 zum Verdicken von Flüssigkeiten in einem pH-Bereich von pH 2,5 bis 6,5, bevorzugt im pH-Bereich von 2,5 bis 5,0.

**Claims**

1. A water-soluble or water-swellable polymer which contains, in random distribution, from 90 to 99.99% by weight

of radicals of the formula (1)

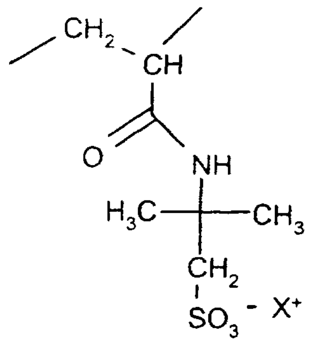

(1)

and from 0.01 to 10% by weight of crosslinking structures which are derived from monomers having at least two olefinic double bonds, where $X^+$ is a cation or a mixture of cations and $X^+$ may comprise not more than 10 mol % of protons ($H^+$), and where the number of radicals of the formula (1) in the polymer must be sufficiently large for the hydrodynamic volume of the polymer in aqueous solution to have a radius of from 10 to 500 nm and a homogeneous, monomodal distribution, wherein

a.) from 90 to 99.99 parts by weight of the 2-acrylamido-2-methylpropanesulfonate of the formula (3) is dissolved or dispersed in a solvent or solvent mixture, where $X^+$ in this case may also comprise up to 100 mol % of protons;

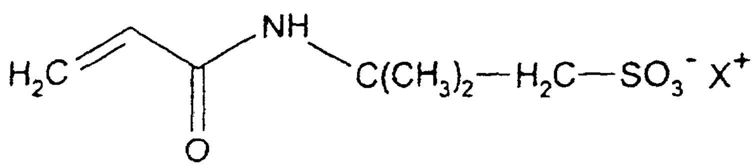

(3)

b.) if required, the solution or dispersion obtained according to a.) is neutralized by means of one or more bases to such an extent that at least 90 mol % of the sulfonic acid are converted into the salt form;

c.) from 0.01 to 10 parts by weight of one or more crosslinking agents having at least two olefinic double bonds are added to the solution obtained according to a.) and b.), and

d.) the polymerization is initiated in a manner known per se by compounds forming free radicals and is carried out at a temperature of from 10 to 150°C,

the choice of the solvent or solvent mixture mentioned in a.) being made so that the polymers obtained are substantially insoluble in the solvent or solvent mixture.

**2.** The water-soluble or water-swellable polymer as claimed in claim 1, which contains 98 - 99.5% by weight of radicals of the formula (1) and from 0.5 to 2% by weight of crosslinking structures which are derived from monomers having at least two olefinic double bonds.

**3.** The water-soluble or water-swellable polymer as claimed in claim 1 and/or claim 2, wherein the crosslinking structures are derived from monomers of the formula (2)

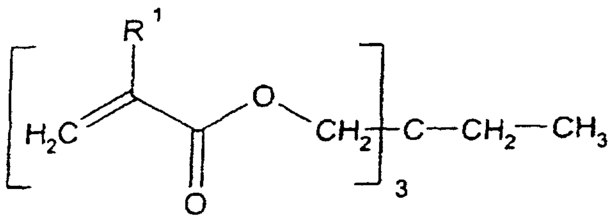

(2)

in which $R^1$ is hydrogen or methyl.

**4.** The water-soluble or water-swellable polymer as claimed in one or more of claims 1 to 3, wherein 90 - 100 mol % of the cations $X^+$ comprise ammonium ions ($NH_4^+$) and from 0 to 10 mol % comprise protons $H^+$.

**5.** The water-soluble or water-swellable polymer as claimed in at least one of claims 1 to 4, wherein a solvent mixture comprising 90 - 100 parts by weight of one or more alcohols and 0 - 10 parts by weight of water is used, the alcohol used preferably being tert-butanol.

**6.** The use of the polymers as claimed in one or more of claims 1 to 5 for thickening liquids.

**7.** The use as claimed in claim 6 for thickening liquids in a pH range of from pH 2.5 to 6.5, preferably in the pH range from 2.5 to 5.0.

**Revendications**

**1.** Polymères solubles ou gonflables dans l'eau qui contiennent en répartition statistique 90 à 99,99 % en poids de groupes de formule générale (1)

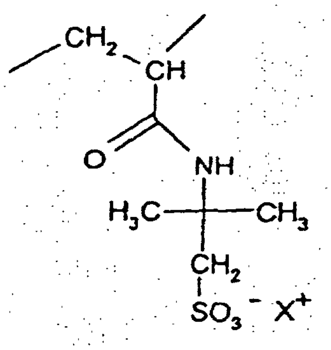

(1)

et 0,01 à 10% en poids de structures réticulées, qui résultent de monomères avec au moins deux doubles liaisons oléfiniques, dans lesquels $X^+$ représente un cation ou un mélange de cations et $X^+$ ne doit pas être constitué de plus de 10 % en mol de protons ($H^+$), et dans lesquels le nombre des groupes de formule générale (1) dans le polymère doit être suffisamment grand pour que le volume hydrodynamique des polymères en solution aqueuse présente un rayon de 10 à 500 nm et une répartition homogène, unimodale, **caractérisés en ce que**

a) on dissout ou on disperse 90 à 99,99 parties en poids du 2-acrylamido-2-méthyl-propane-sulfonate de formule générale (3) dans un solvant ou un mélange de solvants, $X^+$ pouvant dans ce cas être aussi constitué de jusqu'à 100 % en mol de protons

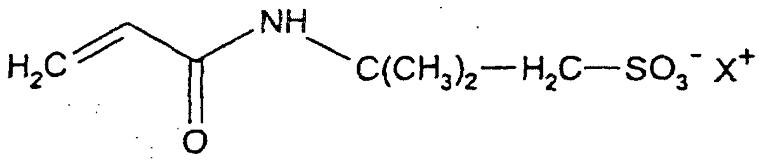 (3)

b) on neutralise éventuellement la solution ou la dispersion obtenue selon a) au moyen d'une ou plusieurs bases, au point qu'au moins 90 % en mol de l'acide sulfonique soit transformé sous forme de sel ;
c) on ajoute à la solution obtenue selon a) et b) 0,01 à 10 parties en poids d'un ou de plusieurs agents de réticulation avec au moins deux doubles liaisons oléfiniques, et
d) on démarre la polymérisation de manière connue en soi au moyen de composés formant des radicaux et on la réalise à une température de 10 à 150 °C,

le choix du solvant ou du mélange de solvants mentionné en a) étant effectué de manière à ce que les polymères obtenus soient largement insolubles dans le solvant ou le mélange de solvants.

2. Polymères solubles ou gonflables dans l'eau selon la revendication 1, **caractérisés en ce qu'**ils contiennent 98-99,5 % en poids de groupes de formule générale (1) et 0,5 à 2 % en poids de structures réticulées, qui résultent de monomères avec au moins deux doubles liaisons oléfiniques.

3. Polymères solubles ou gonflables dans l'eau selon la revendication 1 et/ou la revendication 2, **caractérisés en ce que** les structures réticulées résultent de monomères de formule générale (2)

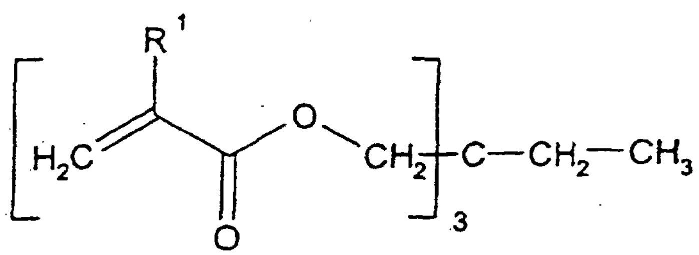 (2)

dans laquelle $R^1$ représente l'hydrogène ou le méthyle.

4. Polymères solubles ou gonflables dans l'eau selon une ou plusieurs des revendications 1 à 3, **caractérisés en ce que** 90-100 % en mol des cations $X^+$ sont constitués d'ions ammonium ($NH_4^+$) et 0 à 10 % en mol de protons $H^+$.

5. Polymères solubles ou gonflables dans l'eau selon au moins une des revendications 1 à 4, **caractérisés en ce qu'**on utilise un mélange de solvants de 90-100 parties en poids d'un ou de plusieurs alcools et 0-10 parties en poids d'eau, dans lequel on utilise comme alcool de préférence le tert.-butanol.

6. Utilisation des polymères selon une ou plusieurs des revendications 1 à 5 pour épaissir des liquides.

7. Utilisation selon la revendication 6 pour épaissir des liquides dans une gamme de pH de 2,5 à 6, 5, de préférence dans la gamme de pH de 2,5 à 5,0.